Europäisches Patentamt

European Patent Office

Office européen des brevets

(11) Veröffentlichungsnummer : **0 020 895**
**B1**

(12) # EUROPÄISCHE PATENTSCHRIFT

(45) Veröffentlichungstag der Patentschrift :
23.05.84

(51) Int. Cl.³ : **C 12 Q    1/56**

(21) Anmeldenummer : **80101898.7**

(22) Anmeldetag : **09.04.80**

(54) **Partielles Thromboplastin.**

(30) Priorität : **14.04.79 DE 2915310**

(43) Veröffentlichungstag der Anmeldung :
**07.01.81 Patentblatt 81/01**

(45) Bekanntmachung des Hinweises auf die Patenterteilung : **23.05.84 Patentblatt 84/21**

(84) Benannte Vertragsstaaten :
**AT BE CH DE FR IT LI NL**

(56) Entgegenhaltungen :
**DE-A- 2 223 235**
**DE-B- 1 189 763**
**DE-C- 2 316 430**
**US-A- 3 486 981**

(73) Patentinhaber : **BEHRINGWERKE AKTIENGESELL-SCHAFT**
**Postfach 1140**
**D-3550 Marburg/Lahn (DE)**

(72) Erfinder : **Schwinn, Horst, Dr.**
**Stümpelstal 27**
**D-3550 Marburg/Lahn (DE)**
Erfinder : **Herchenhan, Bernd**
**Im Brand 33**
**D-3570 Kirchhain (DE)**

(74) Vertreter : **Schüler, Albert, Dr. et al**
**HOECHST AKTIENGESELLSCHAFT** Zentrale Patentabteilung Postfach 80 03 20
**D-6230 Frankfurt am Main 80 (DE)**

EP 0 020 895 B1

**Beschreibung**

Die Erfindung betrifft ein neues partielles Thromboplastin, hergestellt aus pflanzlichen lipidreichen Extrakten, Erdalkalisalzen und Gerbsäurederivaten oder deren Salzen sowie dessen Verwendung.

Zum diagnostischen Nachweis von Blutungsanomalien wird zur katalytischen Unwandlung von Prothrombin in Thrombin eine Thromboplastin genannte Substanz benötigt. Man unterscheidet zwischen kompletten und partiellen Thromboplastinen, wobei das komplette Thromboplastin zum Nachweis der Blutgerinnungsfaktoren II-, V-, VII- und X-bzw. deren Mangel herangezogen wird, während das partielle Thromboplastin zur Diagnose der Hämophilie A (Faktor VIII-Mangel) und Hämophilie B (Faktor IX-Mangel) sowie der seltener auftretenden Faktor XI-, Faktor XII-, Präkallikrein- und Kininogen-Mängel dient.

Es ist bereits bekannt, daß zur Herstellung eines partiellen Thromboplastins Extrakte aus tierischen oder humanen Geweben mit organischen Lösungsmitteln sowie Suspensionen von getrockneten humanen Thrombozyten verwendet werden können. Präparationen dieser Art sind in der US-Patentschrift 3 486 981 (1969) und der deutschen Patentschrift 2 316 430 (Ma 160) beschrieben. Auch pflanzliche Lipid-Fraktionen sind nach W. E. Connor et al. (Proc. Soc. Exp. Biol. Med. *97,* 38 (1958)) als Ausgangsmaterial für ein partielles Thromboplastin brauchbar.

Diese Extrakte ergeben für sich allein noch kein praktisch gebrauchsfähiges Reagenz. Sie sind nur dessen Basis. Ein praktisch einsetzbares partielles Thromboplastin muß nämlich in einem standardisierten Testansatz folgende Werte liefern :

Die Gerinnungszeit eines normalen Citrat-Plasmas muß zwischen 20 und 50 Sekunden liegen ;

Die Gerinnungzeit eines Plasmas mit einem Mangel an mindestens einem der geschilderten Faktoren, muß mindestens das doppelte derjenigen eines Normalplasmas betragen.

Um diesen Anforderungen zu entsprechen, sind sogenannte Aktivatoren notwendig. Als solche dienen beispielsweise wasserunlösliche, oberflächenaktive, anorganische Materialien wie Kieselsäure oder Kaolin oder wasserlösliche organische Säuren, die sich von der Gerbsäure ableiten, wie Ellagsäure, Purpurogallin oder Quercetin bzw. deren Alkali-Salze.

Für die diagnostische Praxis haben alle partiellen Thromboplastine, die einen anorganischen, wasserunlöslichen Aktivator, wie beispielsweise Kaolin, enthalten, den Nachteil, daß sie optisch trübe sind. Sie können deshalb in Koagulometern mit optischer Endpunktanzeige nicht verwendet werden.

Andererseits wurden bisher optisch klare Aktivatoren nur in Verbindung mit tierischen oder humanen Lipid-Extrakten eingesetzt. So können nach O. D. Ratnoff et al. (J. Lab. Clin. Med. *63,* 359 (1964) und USP 3 486 981 Ellagsäure-Salze als Aktivatoren verwendet werden, die in Verbindung mit Extrakten aus tierischem oder humanem Gewebe diagnostisch brauchbare Reagenzien ergeben. Die tierischen oder humanen Extrakte haben aber den Nachteil, daß sie oft nur nach umständlichen Verfahren reproduzierbar hergestellt werden können oder nur in begrenztem Umfang für eine kontinuierliche Produktion zur Verfügung stehen. Es ist deshalb wünschenswert, bei dem ständig steigenden Bedarf an empfindlichen partiellen Thromboplastinen über ein Reagenz mit einem wasserlöslichen, optisch klaren Aktivator zu verfügen, der auf einem pflanzlichen Lipidextrakt basiert.

In Verbindung mit pflanzlichen Lipid-Fraktionen sind bisher allerdings nur anorganische Substanzen als Aktivatoren eingesetzt worden. Es ist bisher nicht bekannt, daß Gerbsäure-Derivate in Verbindung mit pflanzlichen Lipiden ein gebrauchsfähiges partielles Thromboplastin bilden können.

Es ist weiterhin bekannt, daß, abhängig vom Ursprung der Lipid-Extrakte, Qualitätsunterschiede im Nachweis der geschilderten Faktorenmängel auftreten können. So ist z. B. ein Extrakt aus tierischem Gewebe unempfindlicher als ein solcher aus humanem Gewebe. Pflanzliche Lipid-Fraktionen bilden nach M. J. Newlands et al. (Nature *176,* 885 (1955)) Reagenzien, die in ihrer Empfindlichkeit den aus humanem Gewebe hergestellten vergleichbar sind. Pflanzliche Lipide zusammen mit wasserlöslichen Aktivatoren führen jedoch allein noch nicht zu einem geeigneten Reagenz.

Es wurde nun überraschend gefunden, daß ein optisch klares partielles Thromboplastin aus pflanzlichen Lipiden in Verbindung mit einem wasserlöslichen Gerbsäurederivat als Aktivator dann zu einem partiellen Thromboplastin führt, wenn Erdalkali-Metall-Ionen, zweckmäßig in einer optimalen Konzentration, zugefügt werden.

Gegenstand der Erfindung ist deshalb ein partielles Thromboplastin, im wesentlichen bestehend aus Soja-Bohnen-Phospholipid, einem wasserlöslichen Calcium-Salz und Purpurogallin oder Ellagsäure oder deren Alkalisalzen.

Vorzugsweise werden Phospholipid-Präparationen aus der Soja-Bohne verwendet, wie sie kommerziell bezogen werden können. Die Eignung einer solchen Präparation für die Herstellung des erfindungsgemäßen Reagenz kann folgendermaßen überprüft werden :

Man dispergiert die Lipidpräparation zu 0,05 bis 0,1 % in dest. Wasser und setzt anschließend 0,01 bis 0,05 % Ellagsäure zu. Nach 15 min Homogenisieren setzt man soviel Calciumsalz zu, daß eine Endkonzentration des Salzes von $0,1 \cdot 10^{-3}$ bis $0,2 \cdot 10^{-3}$ mol/l resultiert. Wenn diese Lösung im nachstehend beschriebenen Test zur Diagnose von Gerinnungsstörungen mit Normalplasma eine

2

Gerinnungszeit zwischen 20 bis 50 sec ergibt, ist die eingesetzte Lipid-Präparation zur Reagenzherstellung geeignet.

Bei dem Verfahren zur Herstellung des partiellen Thromboplastins kann man beispielsweise wie folgt vorgehen :

Man extrahiert getrocknetes, zerkleinertes Soja-Bohnen-gewebe, mit einer niedrigsiedenden Petrolätherfraktion, wie beispielsweise Hexan oder Heptan, konzentriert den Extrakt durch Verdampfen des organischen Lösungsmittels an und setzt dem Konzentrat einen Überschuß, beispielsweise das 10 fache Volumen, an Azeton zu. Dabei fällt die phospholipidreiche Fraktion aus, die unerwünschten, gerinnungsaktiven Lipide bleiben in Lösung. Der Niederschlag wird gewonnen, gewünschtenfalls durch Umfällen weitergereinigt oder direkt verwendet, nachdem das Fällungsmittel durch Verdampfen in Vakuum entfernt wurde. Solche Präparate können auch im einschlägigen Fachhandel käuflich erworben werden. Das so erhaltene Phospholipid wird in einer Konzentration von 0,01 bis 0,5 % w/v, vorteilhaft 0,05 bis 0,1 % w/v in Wasser vollständig dispergiert, wobei man sich nötigenfalls eines mechanischen Hilfsmittels bedienen kann.

Dazu gibt man 0,005 bis 0,5 % (w/v), vorzugsweise 0,01 bis 0,05 % Ellagsäure in fester, pulverisierter Form unter weiterer mechanischer Bearbeitung, z. B. mit einem hochtourigen Homogenisator und verteilt sie bis zur vollständigen Lösung. Man kann die Ellagsäure auch als konzentrierte Lösung in 0,1 bis 1 n Alkalilauge, beispielsweise in 0,5 n Natronlauge, zusetzen. In diesem Fall sollte allerdings die Phospholipidsuspension vorher mit einer Puffersubstanz, vorteilhaft einer Aminosäure wie Glycin, versetzt werden.

Danach fügt man unter weiterem Rühren soviel einer konzentrierten Calcium Salzlösung, vorzugsweise Calcium-Chlorid-Lösung zu, daß die Endkonzentration an Calcium-Ionen $0,05 \cdot 10^{-3}$ bis $1 \cdot 10^{-3}$ mol/l, vorzugsweise $0,1\text{-}0,2 \cdot 10^{-3}$ mol/l beträgt. Die Calcium-Salzkonzentration ist nach oben hin durch die Löslichkeit des sich bildenden Calcium-Ellagates begrenzt. Der aktivierende Einfluß der Calcium-Ionen und die Abhängigkeit der Gerinnungszeit von der Calcium-Konzentration zeigt die folgende Tabelle am Beispiel von Normalplasma :

| $(Ca^{II}) \cdot 10^{-3}$ mol/l | 0 | 0,05 | 0,1 | 0,2 | 0,5 | 1,0 | 2,0 |
|---|---|---|---|---|---|---|---|
| Gerinnungszeit (sec) | 115 | 49 | 43 | 41 | 41 | 45 | 50 |

Der pH-Wert der fertiggestellten Mischung kann zwischen 6,0 und 8,5 eingestellt werden ; vorteilhaft liegt er bei 7,0-7,8. Die Temperatur während der Herstellung kann zwischen 0 und + 35 °C, vorteilhaft bei 15-25 °C liegen.

Das so hergestellte partielle Thromboplastin kann nach Zusatz von Aminosäuren, wie z. B. Glycin und gewünschtenfalls mit Kohlenhydraten, wie beispielsweise Saccharose, kombiniert gefriergetrocknet werden. Es kann zur Bestimmung der partiellen Thromboplastinzeit oder zur Einzelbestimmung der Faktoren VIII, IX, XI, XII, Präkallikrein oder Kininogen eingesetzt werden.

Der Test zur Diagnose von Störungen des endogenen Gerinnungssystems (partielle Thromboplastinzeit) unter Verwendung des erfindungsgemäßen partiellen Thromboplastins wird folgendermaßen ausgeführt :

Ein Volumenteil der Reagenzlösung wird mit einem Volumenteil normalem Plasma bzw. pathologischem Plasma, in dem die Gerinnungsstörungen qualitativ ermittelt werden sollen, in einem Teströhrchen vermischt und 2 min bei 37 ± 0,5 °C inkubiert. Nach Zusatz von einem Teil einer auf 37 °C vorgewärmten 0,025 molaren Calciumchlorid-Lösung wird die Zeit gemessen, die vom Zusatz der Calciumchlorid-Lösung bis zum Auftreten eines festen Fibringerinnsels vergeht. Weicht die Gerinnungszeit des pathologischen Plasmas von der des Normalplasmas (20 bis 50 sec.) nach längeren Zeiten hin ab, liegt eine Störung in der Aktivität des endogenen Gerinnungssystems vor (Faktor VIII-, XI-, XII-, Präkallikrein- oder Kininogen-Mangel). Voraussetzung für diese Aussage ist, daß andere qualitative Teste (Quick-Wert und Plasmathrombinzeit) normale Gerinnungszeiten zeigen.

Der Test zur Bestimmung der Faktor VIII-, IX-, XI-, XII-, Präkallikrein- oder Kininogen-Aktivität (quantitativ) unter Verwendung des Verfahrensproduktes wird folgendermaßen ausgeführt :

1 Teil der Reagenzlösung wird mit einem Teil eines Faktor VIII-, IX-, XI-, XII-, Präkallikrein- bzw. Kininogen-Mangelplasmas und einem Teil verdünntem Normalplasma bzw. pathologischem Plasma in einem Teströhrchen vermischt. Diese Mischung wird 2 min bei 37 °C gehalten. Nach Zusatz von einem Teil einer auf 37 °C vorgewärmten 0,025 molaren Calciumchlorid-Lösung wird die Zeit bestimmt, die vom Zusatz der Calciumchlorid-Lösung bis zum Auftreten eines festen Gerinnsels verstreicht.

Zur quantitativen Aussage wird die aus dem verdünnten pathologischen Plasma sich ergebende Gerinnungszeit unter Bezugnahme auf eine mit einer Normalplasma-Verdünnungsreihe erzielten Eichkurve abgelesen.

Das erfindungsgemäße partielle Thromboplastin ist in seiner Faktorenempfindlichkeit den aus tierischen oder humanen Geweben hergestellten Thromboplastinen gleichwertig. Dies zeigt folgende Tabelle, in der die Gerinnungszeiten bekannter Thromboplastine mit denen des erfindungsgemäßen Thromboplastins für Normalplasma und Mangelplasma verglichen werden :

| | bekannt | Reagenz bekannt | erfindungsgemäß |
|---|---|---|---|
| Lipidkomponente | tierisch | human | pflanzlich |
| Aktivator | Ellagsäure | Kaolin | Ellagsäure |
| Gerinnungszeit in sec von : | | | |
| Normalplasma | 34 | 44 | 40 |
| Faktor VIII-Mangelplasma | 98 | 112 | 106 |
| Faktor IX-Mangelplasma | 114 | 107 | 124 |
| Faktor XI-Mangelplasma | 118 | 136 | 288 |
| Faktor XII-Mangelplasma | 240 | 223 | 280 |

Die Herstellung des erfindungsgemäßen partiellen Thromboplastins soll an folgendem Beispiel erläutert werden :

Beispiel

5 g Phospholipid aus Soja-Bohnen (Epikuron[(R)] 100 G, Lukas Meyer, Hamburg) werden mittels eines hochtourigen Homogenisators (Janke u. Kunkel AG, Typ 18-10) 30 min bei 2 000 UpM und 15-25 °C in 5 l dest. Wasser bis zur vollständigen Homogenität dispergiert. Danach wird der Homogenisator entfernt, 100 g Glycin unter mäßigem Rühren in fester Form zugegeben und vollständig aufgelöst. Sodann löst man 1,5 g feinpulverisierte Ellagsäure in 50 ml 0,5 h Natronlauge unter Sauerstoffabschluß auf und setzt die tiefviolett gefärbte Lösung unter starkem Rühren in einem Guß der Phospholipid-Glycin-Lösung zu.

Nach 15 min setzt man 30 ml einer 0,025 mol/l Calcium-chlorid-Lösung ebenfalls unter starkem Rühren tropfenweise zu, rührt noch 15 min und mißt den pH-Wert. Dieser beträgt bei dieser Form der Herstellung 7,4. Die Gerinnungszeit einer Frischplasma-Mischung mit dieser Reagenzlösung im beschriebenen Testansatz liegt bei 36 sec, diejenige eines lyophilisierten Standard-Human-Plasmas bei 40 sec.

Die Reagenzlösung wird in Portionen zu 2 ml abgefüllt und lyophilisiert. Man erhält etwa 2 500 Abfüllungen.

**Ansprüche** (für die Vertragsstaaten : BE, CH, DE, FR, IT, LI, NL)

1. Partielles Thromboplastin, im wesentlichen bestehend aus Soja-Bohnen-Phospholipid, einem wasserlöslichen Calcium-Salz und Purpurogallin oder Ellagsäure oder deren Alkalisalzen.

2. Partielles Thromboplastin nach Anspruch 1, dadurch gekennzeichnet, daß es im wesentlichen 0,01 bis 0,5 % w/v Soja-Bohnen-Phospholipid, 0,005 bis 0,5 % w/v Ellagsäure und $0,05 \cdot 10^{-3}$ bis $1,0 \cdot 10^{-3}$ mol/l Calciumionen in wässriger Dispersion enthält.

**Ansprüche** (für den Vertragsstaat AT)

1. Verfahren zur Herstellung eines partiellen Thromboplastins, dadurch gekennzeichnet, daß Soja-Bohnen-Phospholipid in Wasser dispergiert, mit einem wasserlöslichen Calcium-Salz und Purpurogallin oder Ellagsäure oder deren Alkalisalzen versetzt und gegebenenfalls lyophilisiert wird.

2. Verfahren nach Anspruch 1, dadurch gekennzeichnet, daß im wesentlichen das Phospholipid mit soviel Ellagsäure und Calciumionen versetzt wird, daß die wässrige Dispersion 0,01 bis 0,5 % w/v Phospholipid, 0,005 bis 0,5 % w/v Ellagsäure und $0,05 \cdot 10^{-3}$ bis $1 \cdot 10^{-3}$ mol/l Calciumionen enthält.

**Claims** (for the Contracting States : BE, CH, DE, FR, IT, LI, NL)

1. A partial thromboplastin substantially consisting of soybean phospholipid, a water-soluble calcium salt and purpurogallin or ellagic acid or alkali metal salts thereof.

2. Partial thromboplastin as claimed in claim 1, wherein there is contained substantially from 0.01 to 0.5 % w/v of soybean phospholipid, from 0.005 to 0.5 % w/v of ellagic acid and from $0.05 \cdot 10^{-3}$ to $1.0 \cdot 10^{-3}$ mols/l of calcium ions in an aqueous dispersion.

**Claims** (for the Contracting State AT)

1. Process for preparing a partial thromboplastin comprising dispersing soybean phospholipid in water, adding a watersoluble calcium salt and purpurogallin or ellagic acid or alkali metal salts thereof and optionially lyophilizing.

2. Process as claimed in claim 1 substantially comprising adding to the phospholipid sufficient ellagic acid and calcium ions that the aqueous dispersion contains from 0.01 to 0.5 % w/v of phospholipid, from 0.005 to 0.5 % w/v of ellagic acid and from $0.05 \cdot 10^{-3}$ to $1.0 \cdot 10^{-3}$ mols/l of calcium ions.

**Revendications** pour les Etats Contractants : BE, CH, DE, FR, IT, LI, NL)

1. Thromboplastine partielle constituée essentiellement de phospholipide de graines de soja, d'un sel de calcium hydrosoluble et de purpurogalline ou d'acide ellagique ou de ses sels alcalins.

2. Thromboplastine partielle selon la revendication 1, caractérisée en ce qu'elle contient sous forme de dispersion aqueuse essentiellement 0,01 à 0,5 % P/V de phospholipide de graines de soja, 0,005 à 0,5 % P/V d'acide ellagique et $0,05 \cdot 10^{-3}$ à $1 \cdot 10^{-3}$ mole/l d'ions calcium.

**Revendications** (pour l'Etat Contractant AT)

1. Procédé de préparation d'une thromboplastine partielle, caractérisé en ce qu'on disperse un phospholipide de graines de soja dans de l'eau, on l'additionne d'un sel de calcium hydrosoluble et de purpurogalline ou d'acide ellagique ou de ses sels alcalins et le cas échéant on le lyophilise.

2. Procédé suivant la revendication 1, caractérisé en ce qu'essentiellement le phospholipide est additionné d'une quantité d'acide ellagique et d'ions calcium telle que la dispersion aqueuse contient 0,01 à 0,5 % P/V de phospholipide, 0,005 à 0,5 % P/V d'acide ellagique et $0,05 \cdot 10^{-3}$ à $1 \cdot 10^{-3}$ mole/l d'ions calcium.